# FASCICULE DE BREVET EUROPEEN

(11) **EP 1 330 213 B1**
(45) Date de publication et mention de la délivrance du brevet: **04.03.2009**
(21) Numéro de dépôt: 01980615.7
(22) Date de dépôt: 19.10.2001
(51) Int. Cl.: A61F 2/24

(54) **SUPPORT TUBULAIRE DE MISE EN PLACE, PAR VOIE PERCUTANEE, D'UNE VALVE CARDIAQUE DE REMPLACEMENT**
RÖHRENFÖRMIGER TRÄGER ZUM PERKUTANEN EINBRINGEN EINER HERZKLAPPENPROTHESE
TUBULAR SUPPORT FOR SETTING, BY PERCUTANEOUS ROUTE, A SUBSTITUTION HEART VALVE

(30) Priorité: 31.10.2000 FR 0014028
(43) Date de publication de la demande: 30.07.2003
(73) Titulaire: CoreValve, Inc., Irvine, CA 92618 (US)
(72) Inventeur: Séguin, Jacques, Windsor, Berks (GB)
(74) Mandataire: Jeannet, Olivier
(86) Numéro de dépôt international: PCT/FR2001/003258
(87) Numéro de publication internationale: WO 2002/036048

(56) Documents cités:
- WO-A-00/47139
- WO-A1-97/17101
- DE-A- 19 857 887
- US-A- 5 855 601
- US-A- 5 957 949

## Description

La présente invention concerne un support tubulaire de mise en place, par voie percutanée, d'une valve cardiaque de remplacement. Elle concerne également un instrument de mise en place de ce support.

La technique classique de remplacement d'une valve cardiaque consiste à opérer à coeur ouvert. Cette technique oblige à une anesthésie générale, à un large abord, et à un arrêt du coeur impliquant la mise du patient sous circulation extracorporelle. Elle est donc particulièrement lourde à mettre en oeuvre et induit des risques opératoires importants pour le patient.

Pour remédier à cet inconvénient, il a été envisagé d'opérer l'ablation d'une valve cardiaque déficiente par voie percutanée, avec un abord vasculaire périphérique. La demande de brevet n°FR 99 14462 illustre une technique et un dispositif utilisables à cette fin.

En ce qui concerne la mise en place d'une valve cardiaque de remplacement, il a été envisagé de fixer cette valve sur un support présentant une structure en fil ou en réseau de fils, couramment dénommé "stent". Ce support peut être contracté radialement pour pouvoir être introduit dans le corps du patient par voie percutanée, au moyen d'un cathéter, et peut être déployé radialement une fois positionné au niveau du site à équiper de la valve pour assurer ainsi le montage de cette valve. Le brevet n°US 5 411 552 illustre une technique de ce genre.

Les systèmes de "stent" envisagés à ce jour pour la mise en place d'une valve cardiaque enduisent des incertitudes plus ou moins importantes en ce qui concerne leur maintien en position dans les sites à traiter. En particulier, la forme des stents connus n'apparaît pas adaptée à celle de ces sites, dans lesquels la paroi cardiaque s'évase de part et d'autre de l'anneau cardiaque.

En outre, l'anneau cardiaque subsistant après ablation de la valve native peut gêner la mise en place de ces stents.

Ces systèmes connus induisent également, dans certains cas, des problèmes d'étanchéité de la valve de remplacement. En effet, ledit anneau cardiaque subsistant peut présenter une surface plus ou moins irrégulière et plus ou moins calcifiée, qui non seulement amoindrit la qualité de l'appui du stent contre cet anneau mais également peut être à l'origine de fuites entre la valve et cet anneau.

Certains matériels existants présentent par ailleurs des inconvénients en ce qui concerne leur implantation proprement dite. En particulier, ces matériels ne peuvent plus guère être déplacés après déploiement du support même si leur position n'est pas optimale.

La présente invention vise à remédier à ces inconvénients importants.

Son objectif principal est de fournir un support ("stent") procurant une meilleure certitude de maintien en position après implantation.

Un autre objectif de l'Invention est de fournir un support permettant d'éliminer la gêne qu'induisent, au moment de la mise en place de la valve de remplacement, les feuillets valvulaires natifs volontiers calcifiés, épaissis et indurés, ou les restes des feuillets valvulaires après résection valvulaire.

Un autre objectif encore de l'invention est de fournir un support permettant une parfaite étanchéité de la valve de remplacement, même en cas d'un anneau cardiaque subsistant qui présente une surface plus ou moins irrégulière et/ou plus ou moins calcifiée. Le document WO 00/47139 décrit un support tubulaire de mise en place, par voie percutanée, d'une valve cardiaque de remplacement. Le préambule de la revendication 1 est basé sur ce document.

L'invention a également pour objectif de fournir un support dont la position puisse être adaptée et/ou corrigée le cas échéant au moment de l'implantation.

Le support concerné comprend, de manière connue en soi, une structure en fil ou en réseau de fils susceptible d'être contractée radialement pour permettre l'introduction de l'ensemble support-valve dans le corps du patient, au moyen d'un cathéter, et d'être déployée pour permettre la prise d'appui de cette structure contre la paroi du site à équiper de la valve.

Selon l'invention, cette structure comprend :
- une portion axiale de support de valve, présentant une structure en fil ou en réseau de fils propre à recevoir la valve de remplacement montée sur elle, et propre à prendre appui contre l'anneau cardiaque;
- au moins une portion axiale de calage, présentant une structure en fil ou en réseau de fils distincte de la structure de ladite portion axiale de support de valve, et dont au moins une partie présente, à l'état déployé, un diamètre supérieur au diamètre à l'état déployé de ladite portion axiale de support de valve, tel que cette portion axiale de calage est propre à prendre appui contre la paroi bordant ledit anneau cardiaque; et
- au moins un fil de liaison desdites portions, ce ou ces fils étant reliés ponctuellement à ces portions, de manière à ne pas faire obstacle au déploiement desdites portions axiales selon leurs diamètres respectifs.

L'invention fournit ainsi un support en au moins deux portions axiales individualisées l'une par rapport à l'autre en ce qui concerne leur structure, qui sont reliées de manière localisée par au moins un fil ; ce ou ces fils ne font pas obstacle à un déploiement différent de la portion axiale comportant la valve et de la ou des portions axiales de calage.

La présence d'une structure en fil ou en réseau de fils au niveau de la portion axiale de support de valve permet un parfait assemblage de cette valve à cette structure, et la forme ainsi que le diamètre de cette portion axiale peuvent être adaptés à une prise d'appui dans les meilleures conditions contre l'anneau cardiaque subsistant. En particulier, cette portion axiale de support de valve peut présenter une force d'expansion radiale telle qu'elle repousse ("impacte") les feuillets valvulaires volontiers calcifiés, ou les restes des feuillets valvulaires après résection valvulaire sur ou dans les tissus sous-jacents, de sorte que ces éléments ne constituent pas une gêne à la mise en place de la valve de remplacement. Cette structure permet également de supporter d'éventuels moyens d'ancrage du support et/ou d'éventuels moyens d'étanchéité de l'espace existant entre l'anneau cardiaque subsistant et la valve de remplacement, comme indiqué plus loin.

La forme et/ou le diamètre de chaque portion axiale de calage peuvent être adaptés à une prise d'appui dans les meilleures conditions contre la paroi cardiaque située aux abords de l'anneau cardiaque subsistant. Notamment, cette portion axiale de calage peut présenter une forme tubulaire de diamètre constant supérieur à celui de la portion axiale de support de valve, ou une forme tronconique dont le diamètre va en augmentant en s'éloignant de la portion axiale de support de valve.

De préférence, le support tubulaire présente une portion axiale de support de valve en au moins deux parties, dont au moins une est adaptée à supporter la valve et dont au moins une autre est adaptée à repousser les feuillets valvulaires natifs ou les restes des feuillets valvulaires natifs après résection valvulaire, dans ou sur le tissu adjacent afin de rendre cette zone apte à recevoir le support tubulaire.

Cette portion axiale de support de valve élimine la gêne qu'induisent ces éléments valvulaires ou annulaires cardiaques au moment de la mise en place de la valve de remplacement. La force radiale de cette portion axiale de support de valve assure en effet, en impactant tout ou partie du tissu valvulaire dans ou au voisinage de la paroi, une surface plus régulière et plus apte à recevoir l'axe de support de valve. Elle assure également une meilleure solidarisation avec la paroi tout en réduisant le risque de fuite para prothétique.

La portion axiale de support de valve peut notamment comprendre une partie en fil ondulé à larges ondes, pour supporter la valve, et une partie en fil ondulé à ondes réduites, adjacente à ladite partie à larges ondes, présentant une force radiale relativement importante pour permettre de repousser ledit tissu valvulaire contre ou dans la paroi du conduit.

De préférence, le support selon l'invention présente deux portions axiales de calage, l'une étant reliée à une extrémité axiale de ladite portion de support de valve et l'autre à l'autre extrémité axiale de cette même portion de support de valve.

Ces deux portions axiales de calage permettent ainsi un calage du support de part et d'autre de l'anneau cardiaque subsistant, et permettent par conséquent un parfait calage du support dans deux directions opposées par rapport au site traité.

Si nécessaire, par exemple dans le cas où le conduit comprenant la valve présenterait un anévrisme, le support selon l'invention comprend :
- une portion axiale de maintien, propre à prendre appui à l'état déployé contre la paroi du conduit, et
- des fils de liaison tels que précités, reliant ladite portion axiale de support de valve et ladite portion axiale de maintien, ces fils ayant une longueur telle que la portion axiale de maintien soit située après implantation à distance de la portion axiale de support de valve.

Cette distance permet à ladite portion axiale de maintien de prendre appui contre une zone de la paroi du conduit non concernée par une éventuelle affection pouvant exister aux abords de la valve, en particulier un anévrisme. La longueur des fils de liaison peut également être calculée afin d'éviter que la portion axiale de maintien soit au contact des ostia des artères coronaires.

Les portions axiales précitées (de support de valve, de calage, de maintien) peuvent présenter une structure en fil ondulé, en zig-zag ou, de préférence, une structure en mailles avant une forme de losange, les mailles étant juxtaposées dans le sens de la circonférence de ces portions.

Cette dernière structure permet une force radiale adaptée, permettant d'assurer une parfaite prise d'appui dedites portions contre la paroi qui les reçoit.

Le support selon l'invention peut être réalisé en un métal plastiquement déformable. L'instrument de mise en place du support comprend alors un ballonnet présentant une portion axiale d'un diamètre prédéterminé, adaptée à réaliser le déploiement de ladite portion axiale de support de valve, et au moins une portion axiale conformée pour présenter, à l'état gonflé, une section supérieure à celle du conduit à traiter, de manière à réaliser le déploiement de la portion axiale de calage placée sur elle jusqu'à ce que cette portion axiale de calage rencontre la paroi contre laquelle elle doit venir en appui.

Le support selon l'invention peut également être réalisé en un matériau à mémoire de forme, tel qu'un alliage nickel-titane du genre dénommé "NITINOL", pouvant être contracté radialement à une température différente de celle du corps du patient et retrouvant une forme originelle lorsque sa température approche ou atteint celle du corps du patient.

Selon une autre possibilité, le support est réalisé en un matériau à mémoire de forme mais susceptible de déformation plastique, ou comprend des parties en un matériau à mémoire de forme et des parties en un matériau susceptible de déformation plastique, et est conformé de manière telle qu'il puisse être amené, par mémoire de forme ou par déformation plastique, d'un état de contraction à un état de déploiement intermédiaire stable entre l'état de contraction et l'état de déploiement total, puis, par déformation plastique ou par mémoire de forme respectivement, dudit état de déploiement intermédiaire audit état de déploiement total ; dans ledit état de déploiement intermédiaire, le support présente des dimensions telles qu'il reste mobile par rapport au site à traiter.

Le support est ainsi amené au niveau du site à traiter puis est déployé dans ledit état intermédiaire ; sa position peut alors éventuellement être adaptée et/ou corrigée puis le support est amené dans son état de déploiement total.

Le matériau à mémoire de forme mais susceptible de déformation plastique précité peut notamment être un alliage nickel-titane du genre dénommé "NITINOL martensitique", susceptible d'être déformé plastiquement au moyen d'un ballonnet.

Avantageusement, le support selon l'invention comprend des moyens d'ancrage propres à s'insérer dans la paroi du site à traiter, conformés de manière à être mobiles entre une position inactive, dans laquelle ils ne font pas obstacle à l'introduction du support dans le corps du patient, et une position active, dans laquelle ils s'insèrent dans la paroi du site à traiter.

Une parfaite immobilisation du support au niveau du site est ainsi obtenue.

En particulier, ces moyens d'ancrage peuvent présenter la forme d'aiguilles et peuvent être montés sur le support entre des positions de rétractation radiale et des positions de saillie radiale.

Avantageusement, la portion axiale de support de valve comporte, au niveau de sa face extérieure, un moyen d'étanchéité conformé de manière à absorber les irrégularités de surface pouvant exister au niveau ou à proximité de l'anneau cardiaque subsistant.

Ce moyen d'étanchéité peut être constitué par une bande périphérique en un matériau compressible ou par une bande périphérique délimitant une chambre et ayant une structure radialement expansible, cette chambre pouvant recevoir un fluide de gonflage propre à se figer dans un délai déterminé après introduction dans ladite chambre. Ce moyen d'étanchéité peut également comprendre un matériau pouvant être appliqué entre l'anneau cardiaque subsistant et la portion axiale de support de valve, ce matériau étant propre à se figer dans un délai déterminé après cette application. Dans ce cas, ce matériau peut notamment être thermoactivable, par exemple au moyen d'un laser, à travers le ballonnet, ou activable par émission de lumière d'une fréquence déterminée, par exemple au moyen d'un laser à ultra-violets, à travers le ballonnet. Ledit moyen d'étanchéité peut également se présenter sous forme d'un insert gonflable ayant une section transversale en diabolo à l'état gonflé, pouvant être inséré entre l'anneau cardiaque subsistant et la portion axiale de support de valve. Ladite forme en diabolo permet à cet insert de se conformer au mieux aux structures irrégulières adjacentes et d'assurer une meilleure étanchéité.

Pour sa bonne compréhension, l'invention est à nouveau décrite ci-dessous en référence au dessin schématique annexé représentant, à titre d'exemples non limitatifs, plusieurs formes de réalisation possibles du support tubulaire qu'elle concerne.
La figure 1 est une vue en développé de la structure de ce support, selon une première forme de réalisation ;
la figure 2 est une vue en coupe du support à l'état déployé, avec la valve cardiaque qu'il comporte ;
la figure 3 est une vue en bout de ce support à l'état déployé ;
la figure 4 est une vue en bout de ce support à l'état contracté ;
la figure 5 est une vue d'un coeur en coupe partielle, sur lequel une valve cardiaque de remplacement a été mise en place au moyen du support selon l'invention ;
la figure 6 est une vue en développé de la structure du support selon une deuxième forme de réalisation ;
la figure 7 est une vue en développé de la structure du support selon une troisième forme de réalisation ;
la figure 8 est une vue de détail d'une partie de cette structure, à échelle agrandie ;
la figure 9 est une vue du support selon la figure 7, après mise en place dans un conduit corporel présentant un anévrisme ;
la figure 10 est une vue en développé de la structure du support selon une quatrième forme de réalisation ;
la figure 11 est une vue de détail d'une variante de réalisation d'un crochet d'ancrage que comprend le support selon la quatrième forme de réalisation ;
la figure 12 est une vue du même détail, en coupe selon la ligne XII-XII de la figure 11 ;
la figure 13 est une vue similaire à la figure 11, la structure étant dans un état expansé ;
la figure 14 est une vue similaire à la figure 12, la structure étant dans ce même état expansé ;
la figure 15 est une vue partielle d'une portion de structure du support selon une cinquième forme de réalisation ;
la figure 16 est une vue agrandie d'une zone de montage d'une valve prothétique sur cette portion ;
la figure 17 est une vue partielle, en coupe longitudinale, du support selon la première forme de réalisation et d'un anneau cardiaque calcifié ;
la figure 18 est une vue similaire à la figure 17, selon une variante ;
la figure 19 est une vue similaire à la figure 17, selon une autre variante ;
les figures 20 et 21 sont des vues similaires à la figure 17, selon encore une autre variante, dans deux phases de mise en place du support ;
la figure 22 est une vue en coupe longitudinale d'un ballonnet permettant l'expansion de la structure du support représenté aux figures 7 à 9;
la figure 23 est une vue en coupe longitudinale d'un ballonnet selon une variante de réalisation, et
la figure 24 est une vue similaire à la figure 5, montrant un coeur sur lequel une valve cardiaque de remplacement a été mise en place au moyen du support selon l'invention, selon une sixième forme de réalisation.

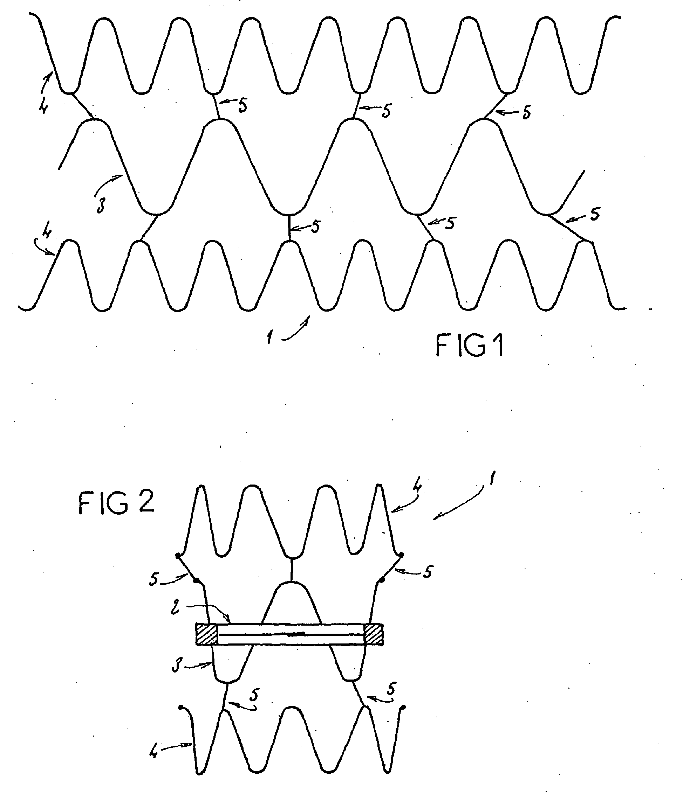

Par simplification, les parties ou éléments de ces différentes formes de réalisation qui sont identiques ou similaires d'une forme de réalisation à une autre sont définis par les mêmes références numériques.Les figures 1 à 4 représentent un support tubulaire 1 de mise en place, par voie percutanée, d'une valve cardiaque 2 de remplacement.

La structure du support 1 comprend une portion médiane 3 comportant la valve 2, deux portions extrémales 4 de calage et des fils 5 de liaison de ces portions 3 et 4. La portion médiane 3 comporte en outre une bande périphérique 6 pourvue d'aiguilles d'ancrage 7 et d'un bandage 8 en matériau compressible.

Ainsi que cela apparaît plus particulièrement sur la figure 1, les portions 3 et 4 sont formées chacune par un fil ondulés, et les fils 5 relient ponctuellement les extrémités des ondulations de la portion 3 à l'extrémité d'une ondulation adjacente d'une portion 4. Les portions 4, vues en développé, ont des longueurs supérieures à la longueur de la portion 3, de sorte que, lorsque les extrémités des fils formant respectivement les portions 3 et 4 sont reliées pour constituer la structure tubulaire du support 1, le diamètre de la portion 3 est inférieur au diamètre des portions 4.

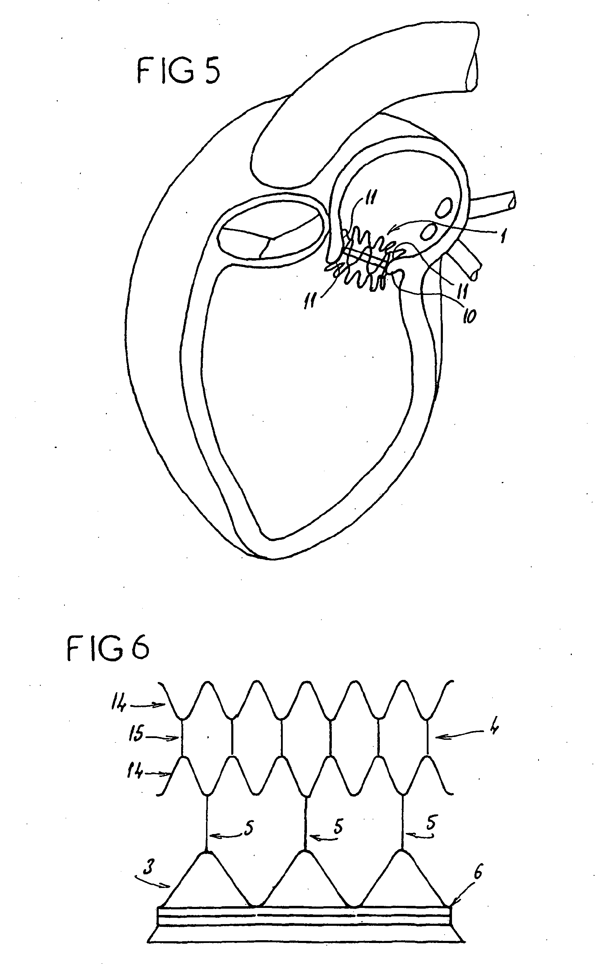

Le diamètre de la portion 3 est tel que cette portion 3 peut, comme le montre la figure 5, prendre appui contre l'anneau cardiaque 10 subsistant après le retrait de la valve native déficiente, tandis que les portions 4 peuvent prendre appui contre les parois 11 bordant l'anneau 10. Ces diamètres respectifs sont de préférence tels que lesdites prises d'appui se font avec une légère contrainte radiale de l'anneau 10 et des parois 11.

La portion 3 présente à l'état déployé un diamètre constant. Les portions 4 peuvent présenter un diamètre constant ou une forme tronconique dont le diamètre va en augmentant en s'éloignant de la portion 3.

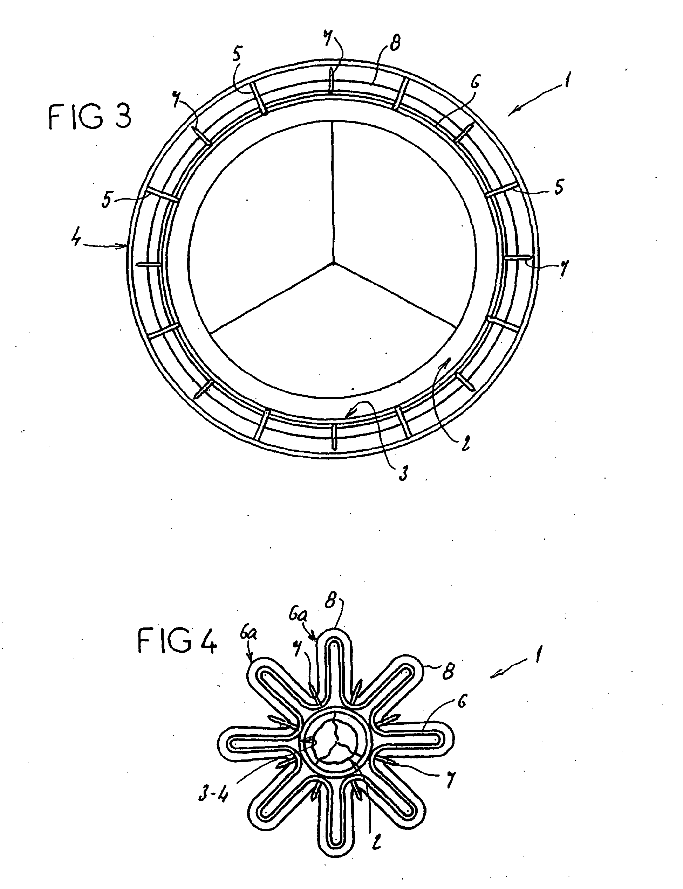

L'ensemble de la structure du support 1 est réalisé en un matériau à mémoire de forme, tel qu'un alliage nickel-titane du genre dénommé "NITINOL". Ce matériau permet à cette structure d'être contractée radialement, comme montré sur la figure 4, à une température différente de celle du corps du patient et de retrouver la forme originelle montrée sur les figures 2 et 3 lorsque sa température approche ou atteint celle du corps du patient. L'ensemble de la structure du support 1 peut également être réalisé en un matériau expandable sur ballonnet comme par exemple en acier inoxydable médical (acier 316 L).

La valve 2 peut être en tissu biologique ou synthétique. Elle est reliée à la portion 3 par des sutures ou par tous autres moyens de fixation appropriés. Elle peut également être moulée sur la portion 3.

La bande 6 est en "NITINOL". Elle est reliée aux ondulations de la portion 3, à mi-hauteur de celles-ci, et comporte les aiguilles 7 au niveau de ses zones reliées à ces ondulations.

Les aiguilles 7 sont constituées par des brins de fil métallique acérés à leurs extrémités libres, et font saillie radialement vers l'extérieur de la bande 6.

Cette dernière peut prendre la forme ondulée visible sur la figure 4 à l'état contracté du support 1 et la forme circulaire visible sur la figure 3 à l'état déployé de ce support 1. Dans sa forme ondulée, la bande 6 forme des ondulations 6a faisant saillie radialement sur l'extérieur du support 1, au-delà des aiguilles 7, de sorte que ces aiguilles 7, dans cette position de rétractation, ne gênent pas l'introduction du support 1 dans un cathéter ni, une fois le support 1 introduit dans le coeur à l'aide de ce cathéter, le déploiement de ce support 1. Le retour de la bande 6 dans sa forme circulaire amène les aiguilles 7 dans une position de déploiement, leur permettant de s'insérer dans l'anneau 10 afin de compléter l'ancrage du support 1.

Le bandage 8 est fixé sur la bande 6. Son matériau compressible lui permet d'absorber les irrégularités de surface pouvant exister au niveau ou à proximité de l'anneau 10 et d'assurer ainsi une parfaite étanchéité de la valve 2.

La figure 6 montre une structure du support 1 comprenant une seule portion 4, reliée à la portion 3 par des fils 5. Cette portion 4 est formée par deux fils 14 ondulés reliés entre eux par des fils 15.

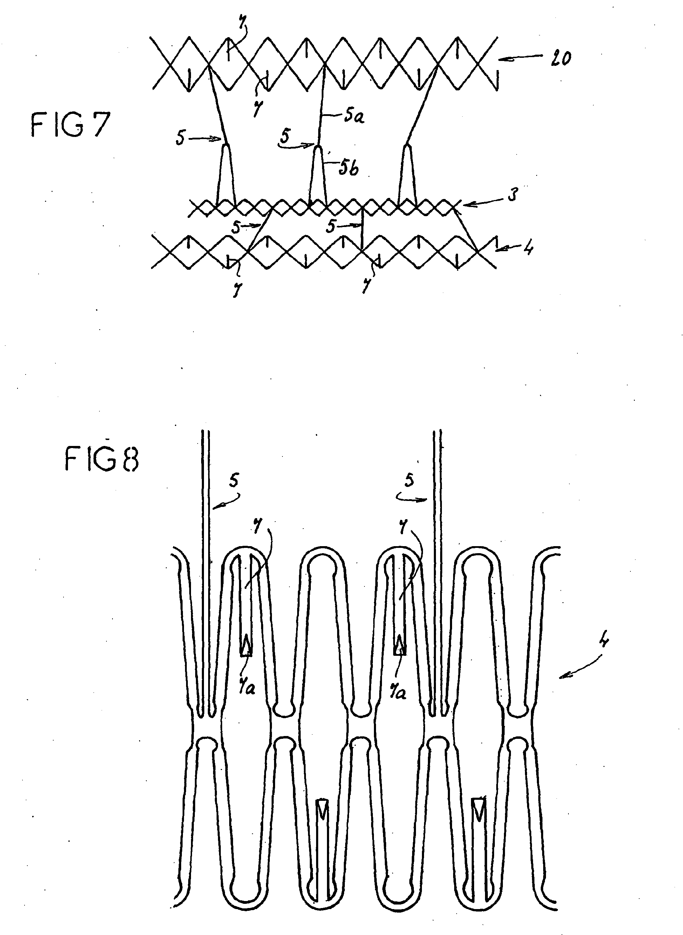

La figure 7 montre une structure du support 1 présentant une portion 3 et une portion 4 reliées par des fils de liaison 5.

Ces portions 3 et 4 ont des structures à mailles en forme de losange, ces mailles étant juxtaposées dans le sens de la circonférence de ces portions et étant reliées les unes aux autres au niveau de deux de leurs angles opposés dans le sens de la circonférence de ces portions 3 et 4. Les fils 5 sont reliés à ces structures au niveau de la zone de jonction de deux mailles consécutives. Ces mailles comportent en outre des crochets d'ancrage 7 s'étendant à travers elles à partir de l'un de leurs angles situés dans le sens longitudinal du support 1.

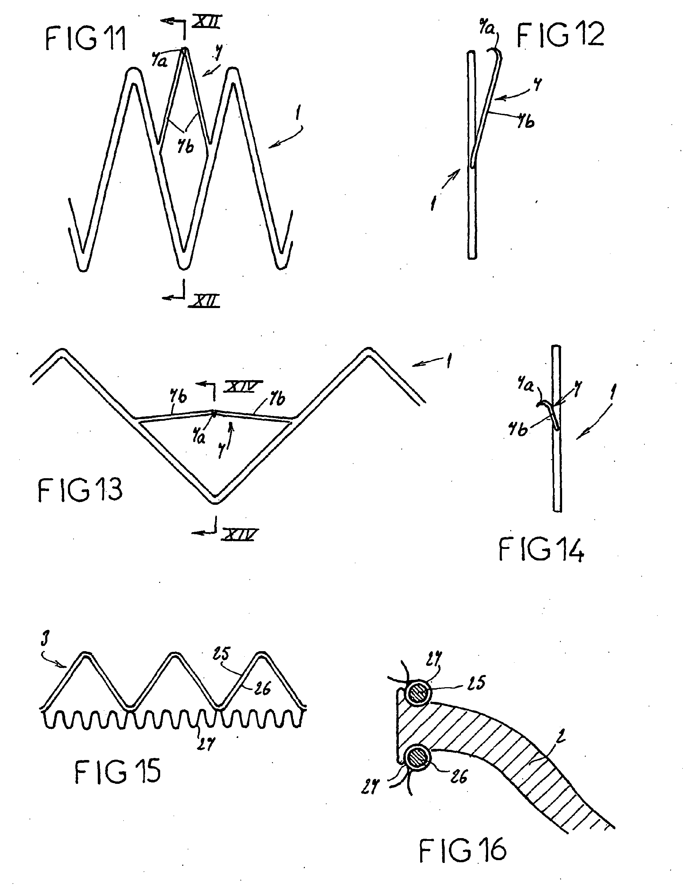

La figure 8 illustre, à échelle agrandie, la structure de cette portion 4 et d'une partie des fils 5, tels que découpés par exemple au laser dans une plaque d'acier inoxydable, et après recourbement des extrémités 7a acérés des crochets 7. Ces derniers ont, vu de profil, une forme telle que montrée sur les figures 12 ou 14.

La structure représentée sur la figure 7 comprend également une portion axiale de maintien 20, ayant une structure identique à celle de la portion 4 mais à maille plus grosses, et trois fils 5 de longueur importante, reliant cette portion 20 à la portion 3. Ces fils 5 ont, du côté de la portion 20, une suture unitaire 5a et, du côté de la portion 3, une structure double 5b. Leur nombre correspond aux trois commissures que forment les trois valvules de la valve 2, ce qui facilite le montage de la valve 2 sur le support 1 ainsi conformé.

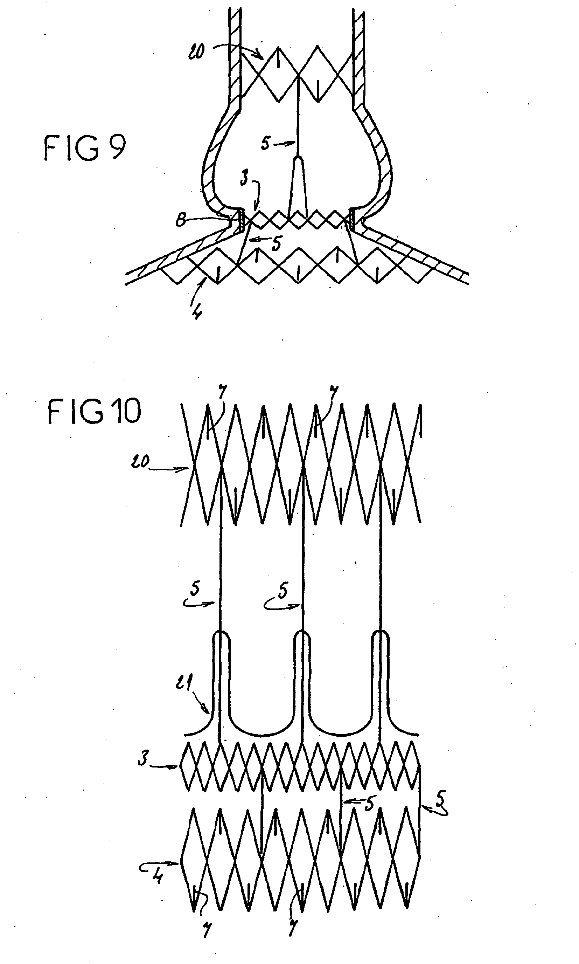

Le support selon la figure 7 est destiné à être utilisé, ainsi cela apparaît sur la figure 9, lorsque le conduit corporel comprenant la valve à remplacer, en particulier l'aorte, présente un anévrisme aux abords de la valve. La longueur des fils 5 reliant les portions 3 et 20 est prévue de manière telle que, après implantation, la portion 20 soit située dans une zone non dilatée dudit conduit corporel, et cette portion 20 est prévue de manière à prendre appui contre la paroi de ce conduit.

La figure 10 montre une structure similaire à la figure 7 mais non expansé, et sinon que les trois fils 5 ont une structure unitaire mais comportent un fil ondulé 21 assurant un appui complémentaire à proximité de la portion 3. Ce fil 21 est conçu pour supporter une valve 2 à trois valvules.

Les figures 11 à 14 montrent une variante de réalisation de la structure des portions 3, 4 ou 20, lorsque cette structure est équipée de crochets 7. Dans ce cas, la structure est en zig-zag et chaque crochet 7 comprend deux branches 7b ; chacune de ces branches 7b est reliée à l'autre branche 7b par une extrémité et à une branche de la structure 1 par son autre extrémité. La zone de jonction des deux branches 7b présente un ergot recourbé 7a d'accrochage.

La figure 15 montre une portion 3 comprenant deux fils ondulés 25, 26 s'étendant l'un à proximité de l'autre et un fil ondulé secondaire 27. Ainsi que le représente la figure 16, les fils 25, 26 peuvent être utilisés pour réaliser l'insertion d'une valve 2 en matériau biologique entre eux et la fixation de cette valve 2 à eux au moyen de sutures 27.

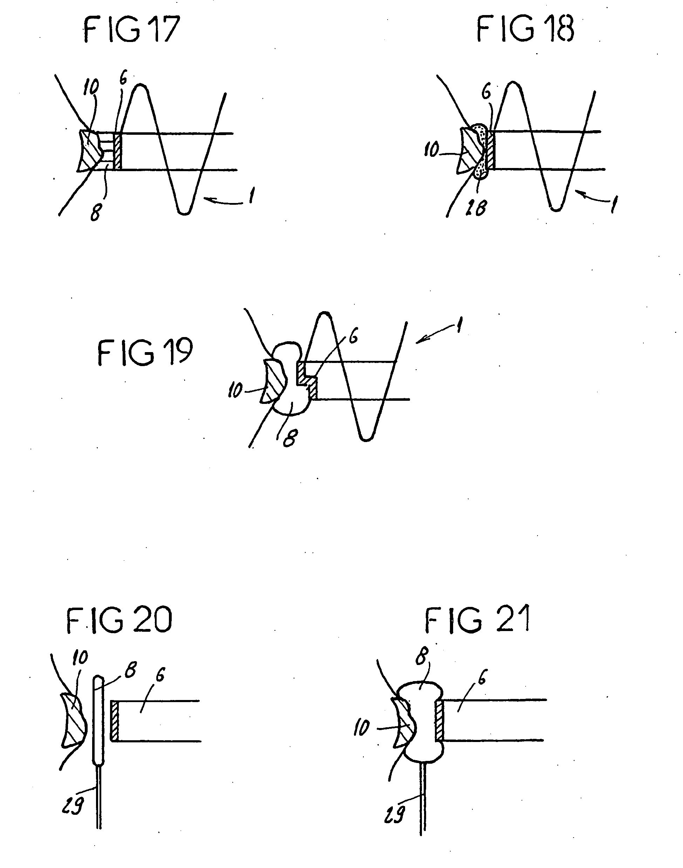

La figure 17 montre une partie du support 1 selon les figures 1 à 5, et la façon dont le matériau compressible constituant le bandage 8 peut absorber les irrégularités de surface pouvant exister au niveau ou à proximité de l'anneau 10, qui résultent de calcifications.

La figure 18 montre un support 1 dont la bande 6 est dépourvue de bandage compressible. Un matériau peut alors être appliqué, au moyen d'une canule appropriée (non représentée), entre l'anneau 10 et cette bande 6, ce matériau étant propre à se figer dans un délai déterminé après cette application.

La figure 19 montre un support 1 dont la bande 6 présente une section en ligne brisée, délimitant, du côté radialement extérieur, un épaulement inférieur. Dans le redan formé par cet épaulement et la paroi circonférientielle adjacente, est logée une bande périphérique 8, gonflable au moyen d'un cathéter (non représenté). Cette bande 8 délimite une chambre et a une structure radialement expansible, telle qu'elle présente en section transversale, à l'état gonflé, deux extrémités élargies faisant saillie de part et d'autre de la bande 6. Cette chambre peut recevoir un fluide de gonflage propre à se figer dans un délai déterminé après introduction dans ladite chambre. Une fois ce matériau figé, le cathéter de gonflage est sectionné.

Les figures 20 et 21 montrent un support 1 dont la bande 6 reçoit un insert gonflable 8 ayant une section transversale en diabolo à l'état gonflé, cet insert 8 pouvant être gonflé au moyen d'un cathéter 29. L'insert 8 est mis en place à l'état dégonflé (figure 20) aux emplacements dans lesquels il existe un espace entre la bande 6 et l'anneau cardiaque subsistant 10. Sa forme en diabolo permet à cet insert 8 (cf. figure 21) de se conformer au mieux aux structures irrégulières adjacentes et d'assurer une meilleure étanchéité.

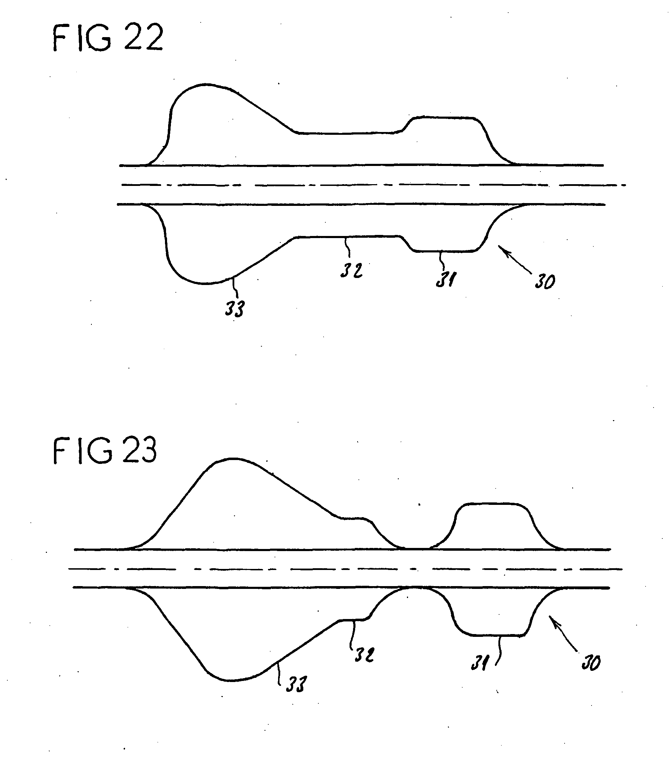

La figure 22 montre un ballonnet 30 permettant de déployer le support 1 selon les figures 7 à 9. Ce ballonnet 30 comprend une portion cylindrique 31 dont le diamètre à l'état gonflé permet l'expansion de la portion de maintien 20, une portion cylindrique 32 de diamètre moindre, adaptée à réaliser l'expansion de la portion 3, et une portion tronconique 33, permettant l'expansion de la portion 4.

Ainsi que montre la figure 23, la portion 32 peut être limitée à ce qui est strictement nécessaire pour déployer la portion 3, ce qui permet de réaliser le ballonnet 30 en deux parties au lieu d'une seule, limitant ainsi le volume de ce ballonnet 30.

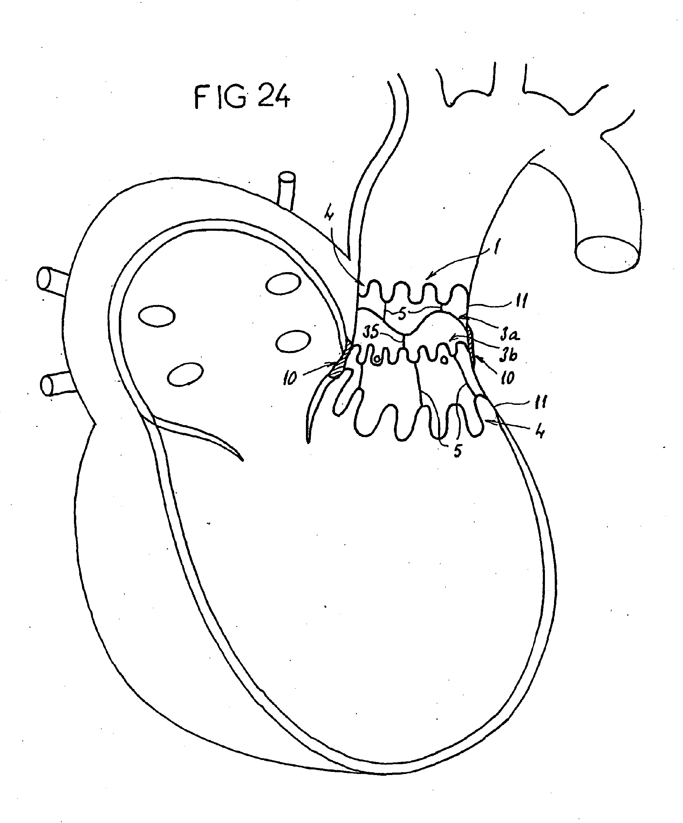

La figure 24 montre un support 1 dont la portion médiane 3 est en deux parties 3a, 3b. La partie 3a est en fil ondulé à larges ondes, pour supporter la valve 2, et la partie 3b, adjacente à ladite partie 3a et reliée à celle-ci par des ponts 35, est en fil ondulé à ondes réduites. Cette partie 3b, par sa structure, présente une force radiale d'expansion relativement élevée et est destinée à être placée en regard de l'anneau 10 afin de repousser les feuillets valvulaires natifs volontiers calcifiés, épaissis et indurés, ou les restes des feuillets valvulaires après résection valvulaire contre ou dans la paroi du conduit.

Cette portion axiale 3a, 3b élimine ainsi la gêne qu'induisent ces feuillets ou résidus de feuillets au moment de la mise en place de la valve 2.

Il apparaît de ce qui précède que l'invention fournit un support tubulaire de mise en place, par voie percutanée, d'une valve cardiaque de remplacement procurant, grâce à ses portions 3 et 4, une parfaite certitude de maintien en position après implantation. Ce support permet également une parfaite étanchéité de la valve de remplacement, même en cas d'un anneau cardiaque présentant une surface plus ou moins irrégulière et/ou plus ou moins calcifiée, et sa position peut être adaptée et/ou corrigée le cas échéant au moment de l'implantation.

Il va de soi que l'invention n'est pas limitée à la forme de réalisation décrite ci-dessus à titre d'exemple mais qu'elle en embrasse au contraire toutes les variantes de réalisation couvertes par les revendications ci-annexées.

## Revendications

1. Support tubulaire de mise en place, par voie percutanée, d'une valve cardiaque de remplacement (2), comprenant une structure en fil ou en réseau de fils susceptible d'être contractée radialement pour permettre l'introduction de l'ensemble support **(1)** - valve (2) dans le corps du patient, au moyen d'un cathéter, et d'être déployée pour permettre la prise d'appui de cette structure contre la paroi du site à équiper de la valve (2), ce support comprenant
- une portion axiale (3) de support de valve (2), présentant une structure en fil ou en réseau de fils propre à recevoir la valve de remplacement (2) montée sur elle, et propre à prendre appui contre l'anneau cardiaque (10);
- au moins une autre portion axiale (4), présentant une structure en fil ou en réseau de fils distincte de la structure de ladite portion axiale (3) de support de valve ;
**caractérisé en ce que** :
- ladite au moins une autre portion axiale est une portion axiale de calage (4), ayant une structure distincte et séparée de la structure de ladite portion axiale (3) de support de valve, au moins une partie de cette portion axiale de calage (4) présentant, à l'état déployé, un diamètre supérieur au diamètre à l'état déployé de ladite portion axiale (3) de support de valve, tel que cette portion axiale de calage (4) est propre à prendre appui contre la paroi bordant ledit anneau cardiaque (10) ; et
- le support tubulaire comprend au moins un fil (5) de liaison desdites portions (3, 4), relié ponctuellement à ces portions (3, 4), de manière à ne pas faire obstacle au déploiement desdites portions axiales (3, 4) selon leurs diamètres respectifs.

2. Support tubulaire selon la revendication 1, **caractérisé en ce qu'**il présente une portion axiale (3) de support de valve en au moins deux parties (3a, 3b), dont au moins une adaptée à supporter la valve (2) et dont au moins une autre adaptée à repousser les feuillets valvulaires natifs ou les restes des feuillets valvulaires natifs après résection valvulaire, dans ou sur le tissu adjacent afin de rendre cette zone apte à recevoir le support tubulaire.

3. Support tubulaire selon la revendication 1 ou la revendication 2, **caractérisé en ce qu'**au moins une portion axiale de calage (4) présente une forme tubulaire de diamètre constant supérieur à celui de la portion axiale (3) de support de valve.

4. Support tubulaire selon la revendication 1 ou la revendication 2, **caractérisé en ce qu'**au moins une portion axiale de calage (4) présente une forme tronconique dont le diamètre va en augmentant en s'éloignant de la portion axiale (3) de support de valve.

5. Support tubulaire selon l'une des revendications 1 à 4, **caractérisé en ce qu'**il comprend :
- une portion axiale de maintien (20), propre à prendre appui à l'état déployé contre la paroi du conduit comprenant la valve, et
- des fils de liaison (5), reliant ladite portion axiale (3) de support de valve et ladite portion axiale de maintien (20), ces fils (5) ayant une longueur telle que la portion axiale de maintien (20) soit située après implantation à distance de la portion axiale (3) de support de valve.

6. Support tubulaire selon l'une des revendications 1 à 5, **caractérisé en ce que** chaque portion axiale de support de valve (3), de calage (4) ou de maintien (20) présente une structure en mailles ayant une forme de losange, les mailles étant juxtaposées dans le sens de la circonférence de cette portion (3, 4, 20).

7. Support tubulaire selon l'une des revendications 1 à 6, **caractérisé en ce qu'**il présente deux portions axiales de calage (4), l'une étant reliée à une extrémité axiale de ladite portion (3) de support de valve et l'autre à l'autre extrémité axiale de cette même portion (3) de support de valve.

8. Support tubulaire selon l'une des revendications 1 à 7, **caractérisé en ce qu'**il est réalisé en un métal plastiquement déformable.

9. Support tubulaire selon l'une des revendications 1 à 7, **caractérisé en ce qu'**il est réalisé en un matériau à mémoire de forme, tel qu'un alliage nickel-titane du genre dénommé "NITINOL", pouvant être contracté radialement à une température différente de celle du corps du patient et retrouvant une forme originelle lorsque sa température approche ou atteint celle du corps du patient.

10. Support tubulaire selon l'une des revendications 1 à 7, **caractérisé en ce qu'**il est réalisé en un matériau à mémoire de forme mais susceptible de déformation plastique, ou comprend des parties en un matériau à mémoire de forme et des parties en un matériau susceptible de déformation plastique, et **en ce qu'**il est conformé de manière telle qu'il puisse être amené, par mémoire de forme ou par déformation plastique, d'un état de contraction à un état de déploiement intermédiaire stable entre l'état de contraction et l'état de déploiement total, puis, par déformation plastique ou par mémoire de forme respectivement, dudit état de déploiement intermédiaire audit état de déploiement total ; dans ledit état de déploiement intermédiaire, le support présente des dimensions telles qu'il reste mobile par rapport au site à traiter.

11. Support tubulaire selon l'une des revendications 1 à 10, **caractérisé en ce qu'**il comprend des moyens d'ancrage (7) propres à s'insérer dans la paroi du site à traiter, conformés de manière à être mobiles entre une position inactive, dans laquelle ils ne font pas obstacle à l'introduction du support (1) dans le corps du patient, et une position active, dans laquelle ils s'insèrent dans la paroi du site à traiter.

12. Support tubulaire selon l'une des revendications 1 à 11, **caractérisé en ce que** ladite portion axiale (3) de support de valve comporte, au niveau de sa face extérieure, un moyen d'étanchéité (8) conformé de manière à absorber les irrégularités de surface pouvant exister au niveau ou à proximité de l'anneau cardiaque (10).

13. Support tubulaire selon la revendication 12, **caractérisé en ce que** le moyen d'étanchéité (8) comprend un bandage périphérique en un matériau compressible.

14. Support tubulaire selon la revendication 12, **caractérisé en ce que** le moyen d'étanchéité (8) comprend un bandage périphérique délimitant une chambre et ayant une structure radialement expansible, cette chambre pouvant recevoir un fluide de gonflage propre à se figer dans un délai déterminé après introduction dans ladite chambre.

15. Support tubulaire selon la revendication 12, **caractérisé en ce que** le moyen d'étanchéité (8) comprend un matériau pouvant être appliqué entre l'anneau cardiaque (10) et la portion axiale (3) de support de valve, ce matériau étant propre à se figer dans un délai déterminé après cette application.

16. Support tubulaire selon la revendication 12, **caractérisé en ce que** le moyen d'étanchéité (8) se présente sous forme d'un insert gonflable ayant une section transversale en diabolo à l'état gonflé, pouvant être inséré entre l'anneau cardiaque (10) et la portion axiale (3) de support de valve.

17. Système comprenant un support selon l'une des revendications 8 ou 10 à 16 et un instrument de mise en place de ce support **caractérisé en ce qu'**il comprend un ballonnet (30) présentant une portion axiale (32) d'un diamètre prédéterminé, adaptée à réaliser le déploiement de ladite portion axiale de support de valve, et au moins une portion axiale (31,33) conformée pour présenter, à l'état gonflé, une section supérieure à celle du conduit à traiter, de manière à réaliser le déploiement de la portion axiale de calage placée sur elle jusqu'à ce que cette portion axiale de calage rencontre la paroi contre laquelle elle doit venir en appui.

## Claims

1. A tubular support for positioning, by percutaneous route, of replacement heart valve (2), which has a structure in the form of a wire or in the form of a network of wires which can be contracted radially in order to make possible the introduction of the assembly comprising support (1) and valve (2) into the body of the patient by means of a catheter, and which can be deployed in order to allow this structure to support the wall of the site which is to be equipped with valve (2), this support (1) comprising
- an axial portion (3) for supporting the valve (2), which has a structure in the form of a wire or in the form of a network of wires suitable for receiving replacement valve (2) mounted thereon, and suitable for bearing against the cardiac ring (10);
- at least one other axial portion (4), which has a structure in the form of a wire or in the form of a network of wires which is distinct from the structure of said axial portion (3) for supporting the valve;
**characterized by** the fact that
- said at least one other axial is an axial wedging portion (4) which is distinct from the structure of said axial portion (3) for supporting the valve, at least a part of this axial wedging portion (4) having, in the deployed state, a diameter greater than the diameter in the deployed state of said axial portion (3) for supporting the valve, such that this axial wedging portion (4) is suitable for supporting the wall bordering said cardiac ring (10); and
- the tubular support comprises at least one wire (5) for connecting said portions (3, 4), this wire or these wires (5) being connected at points to these portions (3, 4), in such a way as not to obstruct the deployment of said axial portions (3, 4) according to their respective diameters.

2. The tubular support according to claim 1, **characterized by** the fact that it comprises an axial portion (3) for supporting the valve in the form of at least two parts (3a, 3b), of which at least one is suitable for supporting the valve (2) and of which at least another is suitable for pushing back the native valve sheets or the residues of the native valve sheets after valve resection, into or onto the adjacent tissue in order to make this region able to receive the tubular support.

3. The tubular support according to claim 1 or 2, **characterized by** the fact that at least one axial wedging portion (4) has a tubular form of constant diameter greater than that of axial portion (3) for supporting the valve.

4. The tubular support according to claim 1 or 2, **characterized by** the fact that at least one axial wedging portion (4) has the form of a truncated cone whose diameter increases away from axial portion (3) for supporting the valve.

5. The tubular support according to one of Claims 1 to 4, **characterized by** the fact that it comprises:
- an axial holding portion (20), suitable for supporting in the deployed state against the wall of the passage containing the valve, and
- connecting wires (5), connecting said axial portion (3) for supporting the valve and said axial holding portion (20), these wires (5) having a length such that axial holding portion (20) is situated after implantation a distance away from axial portion (3) for supporting the valve.

6. The tubular support according to one of Claims 1 to 5, **characterized by** the fact that each axial portion (3) for supporting the valve, axial wedging portion (4) or axial holding portion (20) has a structure in diamond-shaped mesh form, the mesh parts being juxtaposed in the direction of the circumference of this portion (3, 4, 20).

7. The tubular support according to one of Claims 1 to 6, **characterized by** the fact that it has two axial wedging portions (4), one connected to an axial end of said portion (3) for supporting the valve and the other to the other axial end of this same valve support portion (3).

8. The tubular support according to one of Claims 1 to 7, **characterized by** the fact that it is made from a plastically deformable metal.

9. The tubular support according to one of Claims 1 to 7, **characterized by** the fact that it is made from a metal with shape memory, such as the nickel-titanium alloy known as "Nitinol," which can be contracted radially at a temperature different from that of the body of the patient and which regains its original shape when its temperature approaches or reaches that of the body of the patient.

10. The tubular support according to one of Claims 1 to 7, **characterized by** the fact that it is made from a metal with a shape memory but that is plastically deformable, or has parts made from a material with shape memory and parts made from a material that can be plastically deformed, and by the fact that it is shaped in such a way that it can be brought, by shape memory or plastic deformation, from a state of contraction to a stable intermediate state of deployment between the state of contraction and the state of total deployment, and then by plastic deformation or shape memory, as the case may be, from said intermediate state of deployment to said state of total deployment; in said intermediate state of deployment, the support has dimensions such that it remains mobile with respect to the site to be treated.

11. The tubular support according to one of Claims 1 to 10, **characterized by** the fact that it has some anchoring means (7) suitable for being inserted into the wall of the site to be treated, formed in such a way as to be movable between an inactive position, in which it does not obstruct the introduction of support (1) into the body of the patient, and an active position, in which it is inserted in the wall of the site to be treated.

12. The tubular support according to one of Claims 1 to 11, **characterized by** the fact that said axial portion (3) for supporting the valve, at its exterior surface, sealing means (8) formed in such a way as to absorb the surface irregularities which might exist at or near the cardiac ring (10).

13. The tubular support according to claim 12, **characterized by** the fact that sealing means (8) includes a peripheral shell made of a compressible material.

14. The tubular support according to claim 12, **characterized by** the fact that sealing means (8) includes a peripheral shell delimiting a chamber and having a radially expandable structure, this chamber being capable of receiving an inflating fluid suitable for solidification after a predetermined period of time following introduction into said chamber.

15. The tubular support according to claim 12, **characterized by** the fact that sealing means (8) includes a material that can be applied between cardiac ring (10) and axial portion (3) for supporting the valve, this material being suitable for solidification after a predetermined delay following application.

16. The tubular support according to claim 12, **characterized by** the fact that sealing means (8) is present in the form of an inflatable insert with a spool-shaped cross section in the inflated state, which can be inserted between the cardiac ring (10) and axial portion (3) for supporting the valve.

17. The instrument for positioning of the support according to one of claim 8 or 10 to 16, **characterized by** the fact that it includes balloon (30) which has axial portion (32) with a predetermined diameter, suitable for executing the deployment of said axial support portion of the valve, and at least one axial portion (31, 33) shaped in order to have, in the inflated state, a cross section greater than that of the passage to be treated, so as to execute the deployment of the axial wedging portion placed on it until this axial wedging portion encounters the wall it is intended to support.

## Patentansprüche

1. Röhrenförmiger Träger zum perkutanen Einsetzen einer Ersatzherzklappe (2), umfassend eine Struktur aus Draht oder aus einem Drahtnetz, die radial zusammengezogen werden kann, um das Einführen der Konstruktion aus Träger (1) und Klappe (2) in den Körper des Patienten mittels eines Katheters zu ermöglichen, und die entfaltet werden kann, um das Aufstützen dieser Struktur auf der Wand der mit der Klappe (2) auszustattenden Stelle zu ermöglichen, wobei dieser Träger folgendes umfasst:
- einen Axialabschnitt (3) zum Tragen der Klappe (2), der eine Struktur aus Draht oder aus einem Drahtnetz aufweist, die dazu geeignet ist, die darauf eingebaute Ersatzklappe (2) aufzunehmen, und die dazu geeignet ist, sich auf dem Herzring (10) abzustützen ;
- mindestens einen anderen Axialabschnitt (4), der eine Struktur aus Draht oder aus einem Drahtnetz aufweist, die sich von der Struktur des Axialabschnitts (3) zum Tragen der Klappe unterscheidet,
**dadurch gekennzeichnet, dass**:
- der mindestens eine andere Axialabschnitt ein axialer Verkeilungsabschnitt (4) ist, der eine Struktur aufweist, die sich von der Struktur des Axialabschnitts (3) zum Tragen der Klappe unterscheidet und davon getrennt ist, wobei mindestens ein Teil dieses axialen Verkeilungsabschnitts (4) im entfalteten Zustand einen Durchmesser aufweist, der größer ist als der Durchmesser im entfalteten Zustand des Axialabschnitts (3) zum Tragen der Klappe, so dass dieser axiale Verkeilungsabschnitt (4) dazu geeignet ist, sich auf der Wand abzustützen, die den Herzring (20) umrandet; und
- der röhrenförmige Träger mindestens einen Draht (5) zum Verbinden der Abschnitte (3, 4) umfasst, der stellenweise mit diesen Abschnitten (3, 4) verbunden ist, um das Entfalten der Axialabschnitte (3, 4) gemäß ihren jeweiligen Durchmessern nicht zu behindern.

2. Röhrenförmiger Träger nach Anspruch 1, **dadurch gekennzeichnet, dass** er einen Axialabschnitt (3) zum Tragen der Klappe aus mindestens zwei Teilen (3a, 3b) aufweist, von denen mindestens einer in der Lage ist, die Klappe (2) zu tragen, und von denen mindestens ein anderer in der Lage ist, die ursprünglichen Herzklappenblättchen oder die Reste der ursprünglichen Herzklappenblättchen nach der Herzklappenresektion in oder auf dem angrenzenden Gewebe zurückzuschieben, um diesen Bereich zu befähigen, den röhrenförmigen Träger aufzunehmen.

3. Röhrenförmiger Träger nach Anspruch 1 oder Anspruch 2, **dadurch gekennzeichnet, dass** mindestens ein axialer Verkeilungsabschnitt (4) eine Röhrenform mit gleich bleibendem Durchmesser aufweist, der größer ist als derjenige des Axialabschnitts (3) zum Tragen der Klappe.

4. Röhrenförmiger Träger nach Anspruch 1 oder Anspruch 2, **dadurch gekennzeichnet, dass** mindestens ein axialer Verkeilungsabschnitt (4) eine Kegelstumpfform aufweist, deren Durchmesser sich von dem Axialabschnitt (3) zum Tragen der Klappe entfernend vergrößert.

5. Röhrenförmiger Träger nach einem der Ansprüche 1 bis 4, **dadurch gekennzeichnet, dass** er folgendes umfasst:
- einen axialen Halteabschnitt (20), der dazu geeignet ist, sich im entfalteten Zustand auf der Wand des die Klappe umfassenden Kanals abzustützen, und
- Verbindungsdrähte (5), die den Axialabschnitt (3) zum Tragen der Klappe und den axialen Halteabschnitt (20) verbinden, wobei diese Drähte (5) eine Länge aufweisen, so dass der axiale Halteabschnitt (20) sich nach dem Einpflanzen von dem Axialabschnitt (3) zum Tragen der Klappe entfernt befindet.

6. Röhrenförmiger Träger nach einem der Ansprüche 1 bis 5, **dadurch gekennzeichnet, dass** jeder Axialabschnitt zum Tragen der Klappe (3), zum Verkeilen (4) oder zum Festhalten (2) eine Maschenstruktur aufweist, die eine Rautenform hat, wobei die Maschen in Richtung des Umfangs dieses Abschnitts (3, 4, 20) nebeneinander liegen.

7. Röhrenförmiger Träger nach einem der Ansprüche 1 bis 6, **dadurch gekennzeichnet, dass** er zwei axiale Verkeilungsabschnitte (4) aufweist, wobei einer mit einem Axialende des Abschnitts (3) zum Tragen der Klappe und der andere mit dem anderen Axialende desselben Abschnitts (3) zum Tragen der Klappe verbunden ist.

8. Röhrenförmiger Träger nach einem der Ansprüche 1 bis 7, **dadurch gekennzeichnet, dass** er aus einem plastisch verformbaren Metall ausgebildet ist.

9. Röhrenförmiger Träger nach einem der Ansprüche 1 bis 7, **dadurch gekennzeichnet, dass** er aus einem Material mit Formgedächtnis ausgebildet ist, wie etwa einer Nickel-Titan-Legierung von der "NITINOL" genannten Art, das sich bei einer anderen Temperatur als derjenigen des Körpers des Patienten zusammenziehen kann und eine Originalform wieder finden kann, wenn seine Temperatur sich derjenigen des Körpers des Patienten nähert bzw. diese erreicht.

10. Röhrenförmiger Träger nach einem der Ansprüche 1 bis 7, **dadurch gekennzeichnet, dass** er aus einem Material mit Formgedächtnis ausgebildet ist, das jedoch plastisch verformbar ist, oder dass er Teile aus einem Material mit Formgedächtnis und Teile aus einem plastisch verformbaren Material umfasst, und dass er derart ausgestaltet ist, dass er durch Formgedächtnis oder durch plastisches Verformen von einem **zusammengezogenen Zustand in einen beständigen** Zwischenentfaltungszustand zwischen dem zusammengezogenen Zustand und dem ganz entfalteten Zustand gebracht werden kann, und dann jeweils durch **plastisches Verformen oder durch Formgedächtnis von dem** Zwischenentfaltungszustand in den ganz entfalteten Zustand gebracht werden kann; wobei der Träger in dem Zwischenentfaltungszustand derartige Abmessungen aufweist, dass er im Verhältnis zu der zu behandelnden Stelle beweglich bleibt.

11. Röhrenförmiger Träger nach einem der Ansprüche 1 bis 10, **dadurch gekennzeichnet, dass** er Verankerungsmittel (7) umfasst, die dazu geeignet sind, sich in die Wand der zu behandelnden Stelle einzufügen, die derart ausgestaltet sind, dass sie zwischen einer inaktiven Position, in der sie die Einführung des Trägers (1) in den Körper des Patienten nicht behindern, und einer aktiven Position, in der sie sich in die Wand der zu behandelnden Stelle einfügen, beweglich sind.

12. Röhrenförmiger Träger nach einem der Ansprüche 1 bis 11, **dadurch gekennzeichnet, dass** der Axialabschnitt (3) zum Tragen der Klappe an seiner oberen Seite ein Abdichtungsmittel (8) umfasst, das derart gestaltet ist, dass es die oberflächlichen Unebenheiten aufnimmt, die an oder in der Nähe des Herzrings (10) vorhanden sein können.

13. **Röhrenförmiger Träger nach Anspruch 12, dadurch gekennzeichnet, dass** das Abdichtungsmittel (8) ein umlaufendes Verstärkungsband aus einem zusammendrückbaren Material umfasst.

14. Röhrenförmiger Träger nach Anspruch 12, **dadurch gekennzeichnet, dass** das Abdichtungsmittel (8) ein umlaufendes Verstärkungsband umfasst, dass eine Kammer begrenzt und eine radial ausdehnbare Struktur hat, wobei diese Kammer ein Auffüllfluid aufnehmen kann, das dazu geeignet ist, nach einem bestimmten Zeitraum nach der Einführung in die Kammer zu erstarren.

15. **Röhrenförmiger Träger nach Anspruch 12, dadurch gekennzeichnet, dass** das Abdichtungsmittel (8) ein Material umfasst, das zwischen dem Herzring (10) und dem Axialabschnitt (3) zum Tragen der Klappe aufgetragen wird, wobei dieses Material dazu geeignet ist, nach einem bestimmten Zeitraum nach diesem Auftragen zu erstarren.

16. Röhrenförmiger Träger nach Anspruch 12, **dadurch gekennzeichnet, dass** das Abdichtungsmittel (8) in Form eines auffüllbaren Einsatzes vorliegt, der im aufgefüllten Zustand den Querschnitt einer doppelkegeligen Rolle hat und zwischen dem Herzring (10) und dem Axialabschnitt (3) zum Tragen der Klappe eingefügt werden kann.

17. System, umfassend einen Träger nach einem der Ansprüche 8 oder 10 bis 16 und ein Instrument zum Einsetzen dieses Trägers, **dadurch gekennzeichnet, dass** es einen Ballon (30), der einen Axialabschnitt (32) mit einem vorherbestimmten Durchmesser aufweist, der in der Lage ist, das Entfalten des Axialabschnitts zum Tragen der Klappe auszuführen, und mindestens einen Axialabschnitt (31, 33) umfasst, der ausgestaltet ist, um im aufgefüllten Zustand einen Querschnitt aufzuweisen, der größer ist als derjenige des zu behandelnden Kanals, um das Entfalten des axialen Verkeilungsabschnitts auszuführen, der darauf gesetzt ist, bis dieser axiale Verkeilungsabschnitt auf die Wand trifft, auf der er sich abstützen soll.
